(19) Europäisches Patentamt
European Patent Office
Office européen des brevets

(11) EP 3 124 055 B1

(12) **EUROPEAN PATENT SPECIFICATION**

(45) Date of publication and mention
of the grant of the patent:
**20.11.2019 Bulletin 2019/47**

(51) Int Cl.:
*A61L 15/28* (2006.01)          *A61L 15/44* (2006.01)

(21) Application number: **15178911.2**

(22) Date of filing: **29.07.2015**

(54) **BIOLOGICAL FIBER MEMBRANE AND METHOD FOR PREPARING THE SAME**

MEMBRAN AUS BIOLOGISCHEN FASERN UND VERFAHREN ZUR HERSTELLUNG DAVON

MEMBRANE EN FIBRES BIOLOGIQUES ET SON PROCÉDÉ DE PRÉPARATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**01.02.2017 Bulletin 2017/05**

(73) Proprietor: **Harvest Belle Biotech
New Taipei City 248 (TW)**

(72) Inventor: **LU, Chi-Hsiang
248 New Taipei City (TW)**

(74) Representative: **Epping - Hermann - Fischer
Patentanwaltsgesellschaft mbH
Schloßschmidstraße 5
80639 München (DE)**

(56) References cited:
**DE-A1-102012 003 541     US-A- 5 144 021**

- **George M. Garrity ET AL: "Bergey's Manual of
Systematic Bacteriology, Volume Two : The
Proteobacteria Part C The Alpha-, Beta-, Delta-,
and Epsilonproteobacteria" In: "Bergey's Manual
of Systematic Bacteriology, Volume Two : The
Proteobacteria Part C The Alpha-, Beta-, Delta-,
and Epsilonproteobacteria", 1 January 2005
(2005-01-01), Springer, XP055243175, ISBN:
978-0-387-24145-6 vol. 20, pages 72-77, * the
whole document ***

**Description**

**BACKGROUND OF THE INVENTION**

**1. Field of the Invention**

The present invention relates to biological fiber membranes, and more particularly, to a biological fiber membrane applied to the skin.

**2. Description of the Related Art**

In the current medical field, a cotton pad or gauze is mostly used as the dressing for the care of a wound. However, such type of dressing has some drawbacks, for example, a poor antibacterial property, a high possibility of a wound infection, easiness for developing wound adhesion, and difficulty to be removed.

Afterwards, the cotton pads and gauzes have been replaced with non-woven dressings, since the non-woven dressings have the characteristics of better absorbency and being capable of providing a moist environment to aid of the wound repair. However, when the liquid or moisture absorbed by the non-woven dressings is gradually reduced, the issue of wound adhesion is likely to arise.

On the other hand, in addition to the basic living requirements, the modern people pay more attention to cosmetic skin care, especially for the facial care. Hence, the beauty industry focuses on the demands of the facial care, and develops a variety of facial mask products. There are a variety of masks, such as a mud paste-type mask, a tear-peel type mask, a sheet-like mask, and so on.

Although the mud paste-type mask contains some ingredients or minerals for skin care, the mask has to be washed off after application. Hence, the ingredients for skin care are hard to be really absorbed by the skin. Further, because the mud paste-type mask contains more minerals, more preservatives must be added to prevent bacteria from growing in the moist mud paste. The tear-peel type mask has main ingredients, such as, polymer gel, water and alcohol, and promotes the blood circulation of the skin by increasing the epidermal temperature. However, since the tear-peel type mask is no peeled off until being dry, it might cause damage to the sensitive skin during the peeling of the mask. In addition, the tear-peel-type mask does not contain any moisturizing ingredients for the dryness of the mask, such that it is not appropriate for the dry skin. The sheet-like mask is a monolayer sheet absorbed with essence with specific functions, and it can be used for a variety of skin cares by adjusting the ingredients. Although the sheet-like mask does not need to be washed off after application, the mask does not have any cleaning effect. The above sheet-like mask is mostly made of a monolayer of non-woven fabric. For a user to apply the non-woven fabric soaked with the essence, a higher concentration of essence is required due to the rapid water evaporation in the non-woven fabric. Consequently, the essence is wasted, while the problem of water evaporation remains unresolved.

US 5,144,021 A discloses forming a substantially three-dimensionally network of interconnecting fibers from *Acetobactor xylinum* bacteria cultured in the presence of yeast extract. The cellulose production is carried out in a condition involving slow stirring of a mixture at 60 to 65°C.

DE 102012003541 A1 discloses the synthesis of bacterial cellulose by culturing Gluconacetobacter at a pH of 6 to 6.3 in the presence of yeast extract, cellulose and bactopeptone. The structure formed is a regular three-dimensional network.

Therefore, there is still a need to develop a novel dressing product.

**SUMMARY OF THE INVENTION**

In view of the above disadvantages of the prior art, the present invention provides a biological fiber membrane according to claim 1 and a method for the production thereof according to claim 10. Preferred embodiments are set forth in the subclaims.

The biological fiber membrane of the present invention is formed by bacteria of the genus *Gluconacetobacter.* There is a three-dimensional reticular structure bound between the first surface layer and the second surface layer of the biological fiber membrane, such that the moisturizing property of the biological fiber membrane is improved, and thereby being capable of carrying more active ingredients.

**BRIEF DESCRIPTION OF THE DRAWINGS**

FIG. 1 is a schematic diagram of a biological fiber membrane of the present invention;

FIG. 2A shows a scanning electron microscope (SEM) photograph of the three-dimensional reticular structure of the biological fiber membrane of the present invention;

FIG. 2B shows a side view of the biological fiber membrane of the present invention;

FIGs. 3A and 3B show an SEM photograph at 500 X magnification of the biological fiber membrane of the present invention and an SEM photograph at 500 X magnification of the conventional biological fiber membrane, respectively; and

FIG. 4 shows results of a permeability test on the biological fiber membrane of the present invention and the conventional biological fiber membrane, wherein FIG. 4(a) shows the test result of the biological fiber membrane of the present invention, and FIG. 4(b) shows the test result of the conventional biological fiber membrane.

## DETAILED DESCRIPTION OF THE PREFERRED EMBODIMENTS

The following specific examples are used for illustrating the present invention. One skilled in the art can easily conceive the other advantages and effects of the present invention, from the disclosure of the present specification.

It should be noted that all of the drawings depict a structure, proportion, size, etc., are only used to match the specification for a person skilled in the art to understand and reads. It is not intended to limit the conditions which can be implemented in the present invention, such that it is not substantially meaningful technically. Any modification of the structure, change in the proportion or adjustment of the size are be within the scope encompassed in the technical contents disclosed in the present invention, without departing from the spirit of the present invention. At the same time, terms, such as "first," "second," "on" and "a/an" etc., are merely to facilitate the understanding of the descriptions, and should not be construed to limit the implemental scope of the present invention. Any change or adjustment of the relationships is also considered to be within the implemental scope, without any substantial changes to the technical content.

The term "parallel" used herein means the morphology of which a plurality of backbone fibers are in the same direction, such as a longitudinal direction or a width direction.

The present invention provides a biological fiber membrane formed by bacteria of the genus *Gluconacetobacter*, and the biological fiber membrane includes a first surface layer, an opposing second surface layer, and a three-dimensional reticular structure bound between the first surface layer and the second surface layer. The density of the three-dimensional reticular structure is smaller than the density of the first surface layer and the second surface layer.

The biological fiber membrane of the present invention is obtained by culturing microorganisms. It is found in the present invention found that the biological fiber membrane formed by culturing bacteria of the genus *Gluconacetobacter* in a culture medium containing mannitol, peptone, yeast extract and agar has a plurality of biological fibers being wound and combined into a three-dimensional structure.

In an embodiment for producing the biological fiber membrane, the fermentation of the bacterial strain takes place. First of all, a culture medium is provided in a container, and the culture medium contains some known components selected from gelatin, gum arabic, agar, and etc. The culture medium still needs some carbon sources, such as mannitol, glucose, and a combination thereof, and other components, such as peptone and yeast extract. The weight ratio of the carbon source, peptone and yeast extract is in a range from 5:1:1 to 4:1:1. Subsequently, the pH value of the culture medium is preferably controlled at acidic, such as between pH 0.5 to 6. The initial concentration of microorganisms can be controlled in a range of 102 to 105 bacteria/ml. The stationary culturing of the microorganisms is performed at 25 to 28°C for 24 hours to 96 hours. Since a flat container can be used, the three-dimensional reticular structure is flat. After 24 hours to 72 hours, a membrane is taken out, and the biological fiber membrane of the present invention is obtained.

After testing, the thickness of the biological fiber membrane is at least 20 $\mu$m, such as from 20 $\mu$m to 30 $\mu$m, or for example, from 20 $\mu$m to 26 $\mu$m, or from 24 $\mu$m to 26 $\mu$m. The diameter of the biological fiber of the biological fiber membrane of the present invention is from about 20 nm to 100 nm. Further, the amount of the biological fibers per unit area is from 0.005 to 0.008 g/cm$^2$.

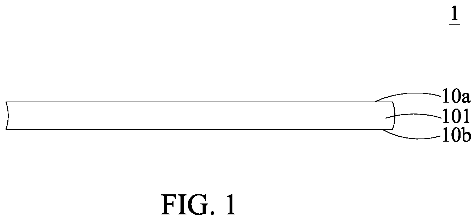

As shown in FIG. 1, a biological fiber membrane 1 of the present invention has a first surface layer 10a and an opposing second surface layer 10b; and a three-dimensional reticular structure 101 bound between the first surface layer 10a and the second surface layer 10b.

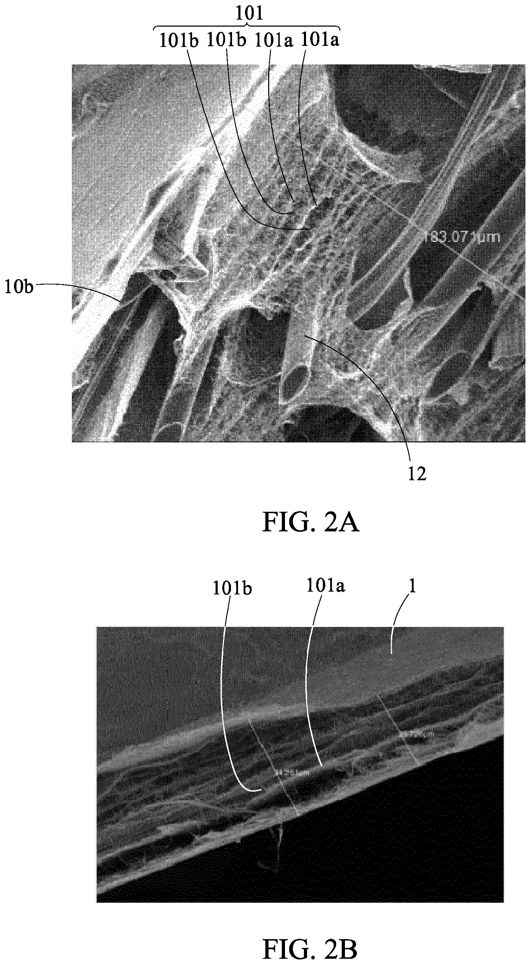

In addition, as shown in FIG. 2A, the three-dimensional reticular structure 101 extends along the second surface layer 10b, and is combined on a cloth membrane fiber 12.

As shown in FIG. 2A, the three-dimensional reticular structure 101 extends to the cloth membrane fiber 12 from the second surface layer 10b, and the three-dimensional reticular structure 101 is composed of a plurality of biological fibers. More particularly, the three-dimensional reticular structure 101 has a plurality of backbone fibers 101a parallel to each other and a plurality of inter-layer fibers 101b interwoven at any two of the adjacent backbone fibers 101a. Therefore, any two of the adjacent backbone fibers 101a are linked to form the three-dimensional reticular structure 101 in the horizontal and vertical directions. Both of the backbone fiber 101a and the inter-layer fiber 101b are biological fibers. As shown in FIG. 2A, the diameter of each of the backbone fibers 101a is greater than the diameter of each of the inter-layer fibers 101b.

As shown in FIG. 2B, a side view of the biological fiber membrane 1 is provided, wherein the biological fiber membrane 1 also has a plurality of backbone fibers 101a, which are parallel to each other or extend along a longitudinal direction or a width direction of the biological fiber membrane 1, and a plurality of inter-layer fibers 101b interwoven with the backbone fibers 101a. In this example, the thickness of the biological fiber membrane 1 is from 20 $\mu$m to 30 $\mu$m. Further, as shown in FIG. 2B, the density of the three-dimensional reticular structure is smaller than the density of the two surface layers of the biological fiber membrane 1. An active ingredient or a drug can be absorbed between the two surface layers, such that a moisturizing effect is provided.

In addition, a substrate can be used for preparing the biological fiber membrane and enable the membrane to be combined temporarily or permanently. Such method can omit the step of flipping the flat three-dimensional reticular structure over in the container. For example, in the example of FIG. 2A, a cloth membrane is used as a substrate.

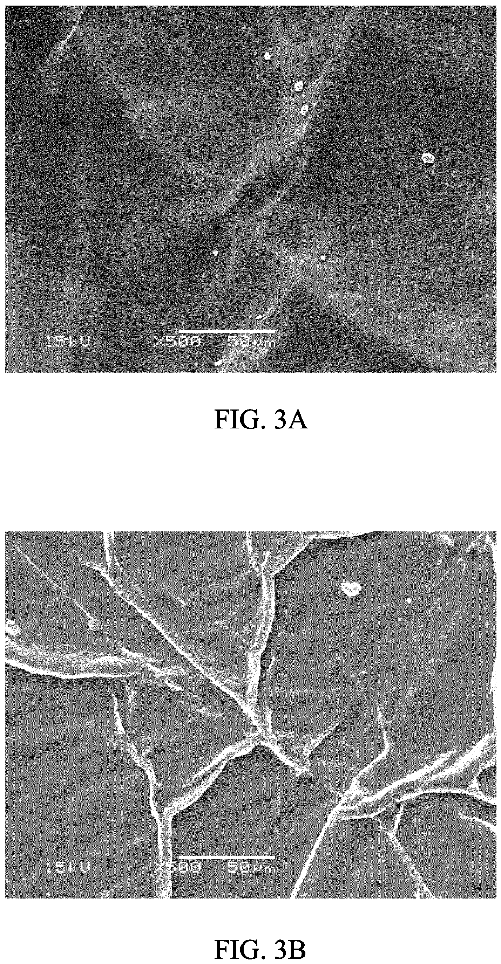

As shown in FIG. 3A and 3B, an SEM photograph at 500 X magnification of the biological fiber membrane of the present invention and an SEM photograph at 500 X magnification of the conventional biological fiber membrane are shown respectively.

As shown in FIG. 3A, the surface of the biological fiber membrane of the present invention is flat. The strips shown in FIG. 3A are fibers of the cloth membrane substrate underneath the biological fiber membrane. Hence, the surface of the biological fiber membrane of the present invention is very flat. On the contrary, the surface of the conventional biological fiber membrane shown in FIG. 3B has many foldings. Therefore, poor attachment of such biological fiber membrane to the skin affects touch, resulting in poor absorbency of a drug or an active ingredient.

**Measurements for the amount of the biological fibers per unit area and absorbency in the biological fiber membrane**

The biological fiber membrane of the present invention was cut into 16 pieces, each with a size of 5 cm x 5 cm. These pieces were dried at 60°C for 10 minutes, and then the dry weight of these pieces were weighed. The result for each of the pieces was obtained by dividing the dry weight by the area of the piece, and the measured amount of the biological fibers per unit area was from 0.005 to 0.008 g/cm$^2$.

Further, these pieces were soaked in water for 5 minutes to weigh the wet weights. The water content per unit area was measured by the following equation:

$$\text{water content per unit area} = (\text{wet weight-dry weight}) \,/\, \text{area}$$

After testing, the water content per unit area of the biological fiber membrane of the present invention was from 0.06 to 0.14 g/cm$^2$.

In comparison, the amount of the biological fibers per unit area of the conventional biological fiber membrane was only 0.001 g/cm$^2$ and the water content of the conventional biological fiber membrane was only 0.04 g/cm$^2$. Therefore, the biological fiber membrane of the present invention is capable of carrying more water or active ingredients, such that the moisturizing effect achieved is better.

**Permeability test on the biological fiber membrane of the present invention**

The biological fiber membrane of the present invention and the conventional biological fiber membrane were placed flat on a substrate, and then the same amount stained essences was added onto the biological fiber membrane of the present invention and the conventional biological fiber membrane, respectively. After 10 seconds, the permeability of the substrate was observed visually.

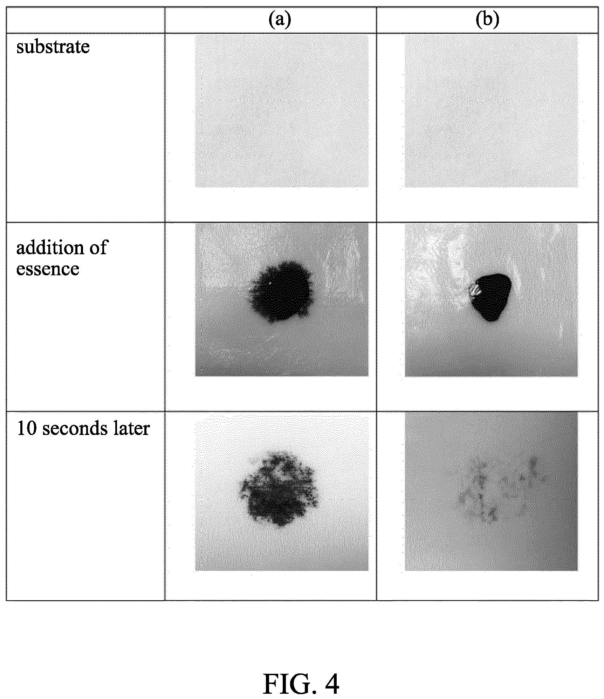

Referring to FIG. 4, FIG. 4(a) shows the test result of the biological fiber membrane of the present invention and FIG. 4(b) shows the test result of the conventional biological fiber membrane. Among these results, when the essences were just added onto the conventional biological fiber membrane, the essences had poor spreading. After 10 seconds, the essences could not penetrate effectively through the substrate. By contrast, the biological fiber membrane of the present invention has better diffusibility and significant permeability.

In summary, the biological fiber membrane of the present invention is provided to apply to the skin. It is particularly useful as a mask. The biological fiber membrane of the present invention further includes an active ingredient or a drug. Before the end of fermentations of a bacterial strain, the active ingredient or drug is added to the three-dimensional reticular structure. Upon completing the preparation of the biological fiber membrane, the active ingredient or drug is included in the membrane. The examples of the active ingredient include humectants, whitening ingredients, anti-wrinkle ingredients, exfoliate ingredients, growth factors and enzymes. The examples of the drug include massage drugs in the form of pastes or liquids, such as massage oil or stress-relieving oil. Because the biological fiber membrane of the present invention has a three-dimensional reticular structure, the active ingredient or drug is not easily exposed. After applying the biological fiber membrane of the present invention to the body or skin and pressing the biological fiber membrane by massage or other contact devices, the release of the active ingredient or drug is facilitated. In addition, the release of the active ingredient or drug is also facilitated by heating.

## Claims

1. A biological fiber membrane formed by culturing bacteria of the genus Gluconacetobacter in a culture medium having mannitol, peptone, yeast extract and agar, comprising:

   a first surface layer and an opposing second surface layer; and a three-dimensional reticular structure bound between the first surface layer and the second surface layer, wherein the density of the three-dimensional reticular structure is lower than the density of the first surface layer and the second surface layer, wherein the three-dimensional reticular structure has a plurality of backbone fibers parallel to each other and a plurality of inter-layer fibers interwoven at any two of adjacent backbone fibers, and the diameter of each of the plurality of backbone fibers is greater than or equal to the diameter of each of the plurality of inter-layer fibers, and wherein the plurality of backbone fibers extend along a longitudinal direction or a width direction of the biological fiber membrane, and wherein the water content per unit area of the biological fiber membrane is from 0.06 to 0.14 $g/cm^2$.

2. The biological fiber membrane of claim 1, wherein the three-dimensional reticular structure is composed of a plurality of biological fibers.

3. The biological fiber membrane of claim 1, wherein the plurality of backbone fibers and the plurality of inter-layer fibers are biological fibers.

4. The biological fiber membrane of claim 1, further comprising an active ingredient or a drug.

5. The biological fiber membrane of claim 4, wherein the active ingredient is selected from the group consisting of a humectant, a whitening ingredient, an anti-wrinkle ingredient, an exfoliating ingredient, a growth factor and an enzyme.

6. The biological fiber membrane of claim 2, wherein the amount of the biological fibers per unit area in the biological fiber membrane is from 0.005 $g/cm^2$ to 0.008 $g/cm^2$.

7. The biological fiber membrane of claim 1 having a thickness of at least 20 $\mu$m.

8. The biological fiber membrane of claim 7, wherein the thickness is from 20 $\mu$m to 30 $\mu$m.

9. The biological fiber membrane of claim 2, wherein the diameter of each of the biological fiber is from 20 nm to 100 nm.

10. A method for preparing the biological fiber membrane of any one of the preceding claims, comprising:

    providing a flat container having a culture medium comprising mannitol and agar, wherein the culture medium has a carbon source, a peptone and a yeast extract at a weight ratio from 5:1:1 to 4:1:1; and stationarily culturing bacteria of the genus Gluconacetobacter in the culture medium at 25 to 28°C for 24 hours to 96 hours, wherein the pH value of the culture medium is from 0.5 to 6.

## Patentansprüche

1. Membran aus biologischen Fasern, die durch Züchtung von Bakterien der Gattung Gluconacetobacter in einem Nährmedium mit Mannitol, Pepton, Hefeextrakt und Agar ausgebildet ist, umfassend:

   eine erste Oberflächenschicht und eine entgegengesetzte zweite Oberflächenschicht; und eine dreidimensionale retikuläre Struktur, die zwischen die erste Oberflächenschicht und die zweite Oberflächenschicht eingebunden ist, wobei die Dichte der dreidimensionalen retikulären Struktur geringer als die Dichte der ersten Oberflächenschicht und der zweiten Oberflächenschicht ist, wobei die dreidimensionale retikuläre Struktur eine Vielzahl zueinander paralleler Gerüstfasern und eine Vielzahl von Zwischenschichtfasern, die an zwei beliebigen angrenzenden Gerüstfasern verwoben sind, aufweist, und der Durchmesser von jeder der Vielzahl von Gerüstfasern größer oder gleich dem Durchmesser von jeder der Vielzahl von Zwischenschichtfasern ist, und wobei sich die Vielzahl von Gerüstfasern entlang einer Längsrichtung oder einer Breitenrichtung der Membran aus biologischen Fasern erstreckt, und wobei der Wassergehalt pro Flächeneinheit der Membran aus biologischen Fasern 0,06 bis 0,14 g/cm$^2$ beträgt.

2. Membran aus biologischen Fasern nach Anspruch 1, wobei die dreidimensionale retikuläre Struktur aus einer Vielzahl biologischer Fasern besteht.

3. Membran aus biologischen Fasern nach Anspruch 1, wobei die Vielzahl von Gerüstfasern und die Vielzahl von Zwischenschichtfasern biologische Fasern sind.

4. Membran aus biologischen Fasern nach Anspruch 1, ferner umfassend einen Wirkstoff oder ein Arzneimittel.

5. Membran aus biologischen Fasern nach Anspruch 4, wobei der Wirkstoff aus der Gruppe ausgewählt ist, die aus Feuchthaltemittel, einem aufhellenden Inhaltsstoff, einem Antifaltenwirkstoff, einem Peeling-Inhaltsstoff, einem Wachstumsfaktor und einem Enzym besteht.

6. Membran aus biologischen Fasern nach Anspruch 2, wobei die Menge der biologischen Fasern pro Flächeneinheit in der Membran aus biologischen Fasern 0,005 g/cm$^2$ bis 0,008 g/cm$^2$ beträgt.

7. Membran aus biologischen Fasern nach Anspruch 1, die eine Dicke von mindestens 20 μm aufweist.

8. Membran aus biologischen Fasern nach Anspruch 7, wobei die Dicke 20 μm bis 30 μm beträgt.

9. Membran aus biologischen Fasern nach Anspruch 2, wobei der Durchmesser jeder der biologischen Fasern 20 nm bis 100 nm beträgt.

10. Verfahren zur Herstellung einer Membran aus biologischen Fasern nach einem der vorhergehenden Ansprüche, umfassend:

Bereitstellen eines flachen Behälters mit einem Nährmedium, das Mannitol und Agar umfasst, wobei das Nährmedium eine Kohlenstoffquelle, ein Pepton und ein Hefeextrakt mit einem Gewichtsverhältnis von 5:1:1 bis 4:1:1 aufweist; und
stationäres Züchten von Bakterien der Gattung Gluconacetobacter in dem Nährmedium bei 25 bis 28°C für 24 Stunden bis 96 Stunden, wobei der pH-Wert des Nährmediums 0,5 bis 6 beträgt.

**Revendications**

1. Membrane en fibres biologiques formée par culture de bactéries du genre Gluconacetobacter dans un milieu de culture contenant du mannitol, de la peptone, de l'extrait de levure et de l'agar, comprenant :

une première couche de surface et une deuxième couche de surface opposée ; et une structure réticulaire tridimensionnelle liée entre la première couche de surface et la deuxième couche de surface, la densité de la structure réticulaire tridimensionnelle étant inférieure à la densité de la première couche de surface et de la deuxième couche de surface, la structure réticulaire tridimensionnelle possédant une pluralité de fibres de squelette parallèles les unes aux autres et
une pluralité de fibres inter-couches entrelacées au niveau de deux fibres de squelette adjacentes quelconques et le diamètre de chacune de la pluralité de fibres de squelette étant supérieur ou égal au diamètre de chacune de la pluralité de fibres inter-couches et la pluralité de fibres de squelette s'étendant le long d'une direction longitudinale ou d'une direction de largeur de la membrane en fibres biologiques et la teneur en eau par surface unitaire de la membrane en fibres biologiques étant de 0,06 à 0,14 g/cm$^2$.

**2.** Membrane en fibres biologiques selon la revendication 1, la structure réticulaire tridimensionnelle étant composée d'une pluralité de fibres biologiques.

**3.** Membrane en fibres biologiques selon la revendication 1, la pluralité de fibres de squelette et la pluralité de fibres inter-couches étant des fibres biologiques.

**4.** Membrane en fibres biologiques selon la revendication 1, comprenant en outre un principe actif ou un médicament.

**5.** Membrane en fibres biologiques selon la revendication 4, le principe actif étant choisi dans le groupe constitué par un humectant, un ingrédient de blanchiment, un ingrédient antirides, un ingrédient exfoliant, un facteur de croissance et une enzyme.

**6.** Membrane en fibres biologiques selon la revendication 2, la quantité des fibres biologiques par surface unitaire dans la membrane en fibres biologiques étant de 0,005 g/cm$^2$ à 0,008 g/cm$^2$.

**7.** Membrane en fibres biologiques selon la revendication 1, présentant une épaisseur d'au moins 20 $\mu$m.

**8.** Membrane en fibres biologiques selon la revendication 7, l'épaisseur étant de 20 $\mu$m à 30 $\mu$m.

**9.** Membrane en fibres biologiques selon la revendication 2, le diamètre de chacune des fibres biologiques étant de 20 nm à 100 nm.

**10.** Procédé de préparation de la membrane en fibres biologiques selon l'une quelconque des revendications précédentes, comprenant :

l'utilisation d'un récipient plat contenant un milieu de culture comprenant du mannitol et de l'agar, le milieu de culture contenant une source de carbone, une peptone et un extrait de levure dans un rapport pondéral de 5:1:1 à 4:1:1 ; et
la culture stationnaire de bactéries du genre Gluconacetobacter dans le milieu de culture à 25 jusqu'à 28°C pendant 24 heures à 96 heures, la valeur de pH du milieu de culture étant de 0,5 à 6.

1

10a
101
10b

FIG. 1

101
101b 101b 101a 101a

10b

12

FIG. 2A

101b     101a     1

FIG. 2B

FIG. 3A

FIG. 3B

|  | (a) | (b) |
|---|---|---|
| substrate | | |
| addition of essence | | |
| 10 seconds later | | |

# FIG. 4

**EP 3 124 055 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5144021 A
- DE 102012003541 A1